**Europäisches Patentamt**

**European Patent Office**

**Office européen des brevets**

(19)

(11) Veröffentlichungsnummer: **0 204 218**
**B1**

(12) **EUROPÄISCHE PATENTSCHRIFT**

(45) Veröffentlichungstag der Patentschrift:
**16.08.89**

(51) Int. Cl.⁴: **A61M 25/00**

(21) Anmeldenummer: **86106933.4**

(22) Anmeldetag: **22.05.86**

(54) **Ballonkatheter.**

(30) Priorität: **31.05.85 DE 3519626**

(43) Veröffentlichungstag der Anmeldung:
**10.12.86 Patentblatt 86/50**

(45) Bekanntmachung des Hinweises auf die Patenterteilung:
**16.08.89 Patentblatt 89/33**

(84) Benannte Vertragsstaaten:
**DE FR GB IT**

(56) Entgegenhaltungen:
**US-A- 2 927 584**
**US-A- 3 635 223**
**US-A- 3 638 655**
**US-A- 3 895 637**
**US-A- 4 465 072**

(73) Patentinhaber: **Stöckert Instrumente GmbH,**
**Osterwaldstrasse 10, D-8000 München 40(DE)**

(72) Erfinder: **Saubert, Hans, Am Hügel 6,**
**D-8642 Ludwigsstadt/Ofr.(DE)**
Erfinder: **Saubert, Gerd, Am Hügel 6,**
**D-8642 Ludwigsstadt/Ofr.(DE)**

(74) Vertreter: **Hoffmann, Klaus, Dr. rer. nat. et al, Hoffmann .**
**Eitle & Partner Patentanwälte**
**Postfach 81 04 20 Arabellastrasse 4,**
**D-8000 München 81(DE)**

## Beschreibung

Die Erfindung betrifft einen Ballonkatheter mit einer aus dehnbarem Material bestehenden Ballonhülle. Derartige Ballonkatheter werden in der Medizin auf verschiedene Weise angewandt. So werden sie z.B. zum intravaskulären und dauerhaften Verschließen von Gefäßverbindungen und Herzscheidewanddefekten, zum Schließen von Öffnungen in Gefäßen oder anderen menschlichen Organen, zum Entfernen von Blutgerinseln aus Gefäßen, aber auch zu andersartigen Zwecken, beispielsweise zur Abdichtung von Tubussen für künstliche Beatmung gegenüber der Luftröhre und ähnlichem verwendet.

In vielen der Anwendungsfälle besteht der Wunsch, daß der Ballon nach seinem Aufblasen einen festen Sitz in seiner Umgebung hat, in welcher er plaziert werden soll. Zu diesem Zweck ist es bei derartigen Ballonkathetern bereits bekannt, die äußere Oberfläche der Ballonhülle mit von ihr abstehenden Flocken zu versehen, die aus Baumwolle, Wolle, Nylon oder ähnlichen synthetischen Fasern bestehen und sich an den Wandungen der Gefäße oder anderen menschlichen Organen haftend anlegen oder festkrallen sollen. Da derartige Flockfasern aber in der Regel sehr weich und biegsam sind, ist trotz dieser von der Ballonoberfläche abstehenden Flocken ein fester Sitz des Ballonkatheters in Gefäßen oder anderen Organen des menschlichen oder tierischen Körpers nicht immer ausreichend sichergestellt (US-A 2 927 584).

Der Erfindung liegt daher die Aufgabe zugrunde, mit einer andersartigen Ausbildung der äußeren Oberfläche der Ballonhülle mit größtmöglicher Sicherheit einen festen Sitz des Ballonkatheters in seiner Umgebung zu schaffen, in der er plaziert werden soll. Außerdem soll seine Oberfläche so beschaffen sein, daß ein Festhaften von Partikelchen, wie Blutgerinsel od. dgl., aus Gefäßen oder anderen Organen des menschlichen oder tierischen Körpers an seiner Oberfläche möglich ist. Der Ballonkatheter soll sich außerdem gut an seine Umgebung anschmiegen bzw. mit ihn umgebenden Teilen des menschlichen oder tierischen Körpers verbinden.

Diese Aufgabe wird erfindungsgemäß dadurch gelöst, daß die Ballonhülle eine unebene äußere Oberfläche mit kraterartigen Vertiefungen von 0,1 bis 0,5 mm, vorzugsweise mindestens 0,3 mm, Tiefe hat. Diese unebene Oberfläche kann Hinterschneidungen, Überhänge oder krallenförmige Erhöhungen haben.

Die vorgenannte unregelmäßig unebene Oberfläche kann sich über die gesamte äußere Oberfläche der Ballonhülle, aber auch nur über einen Teil derselben erstrecken. Sie kann von einer Beschichtung aus dehnbarem Material gebildet sein, welche auf die eigentliche dehnbare luftdichte Ballonhülle aufgebracht ist. Diese Beschichtung kann porige oder schaumförmige Struktur, insbesondere mit offenen Poren haben. Die Beschichtung kann z.B. aus Latex, aus Silikon, aus Polyurethan od. dgl. bestehen.

In der Zeichnung ist ein besonders vorteilhaftes Ausführungsbeispiel des erfindungsgemäßen Ballonkatheters dargestellt, welches im folgenden näher beschrieben wird:

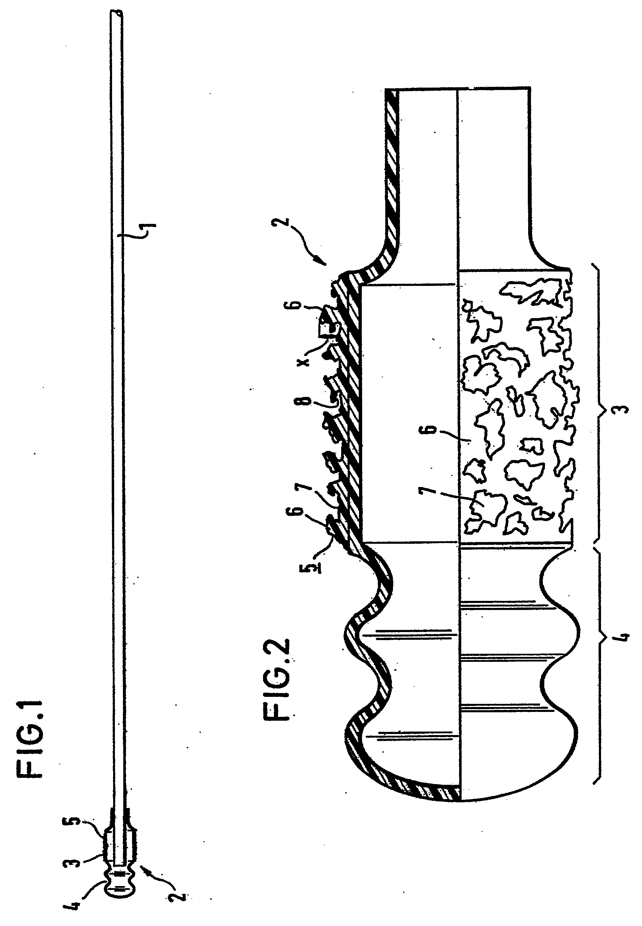

Fig. 1 zeigt dieses Ausführungsbeispiel im Längsschnitt durch den Katheter,

Fig. 2 zeigt den Ballon dieses Katheters in starker Vergrößerung, teilweise in Seitenansicht, teilweise im Längsschnitt.

Der erfindungsgemäße Katheter trägt am vorderen Ende seines Katheterschlauches 1 einen aus elastischem Material gebildeten Ballon 2, der durch Aufstecken auf den Katheterschlauch 1 mit diesem fest verbunden ist. Die Ballonhülle hat einen zylindrischen Teil 3 und einen ziehharmonikaförmig gefalteten Teil 4, um beim Aufblasen ihre Aufweitung zu erleichtern. Die Ballonhülle besteht in üblicher Weise aus dehnbarem Material, wie Gummi, dehnbarem Kunststoff od.dgl. In ihrem zylindrischen Teil 3 ist die äußere Oberfläche der Ballonhülle mit einer Beschichtung 5 versehen, die ebenso wie die Ballonhülle 2 selbst aus dehnbarem Material, wie Latex, Silikon, natürlichem oder synthetischem Gummi, Polyurethan od.dgl. bestehen kann. Die äußere Oberfläche dieser Beschichtung hat eine unregelmäßig unebene naturschwammförmige Struktur mit unregelmäßigen Erhöhungen 6 und Vertiefungen 7. Die Vertiefungen haben kraterähnliche Form mit einer Tiefe x von 0,2-0,5 mm, vorzugsweise jedoch mindestens 0,3 mm. Die unebene Oberfläche hat Kanten, Spitzen und Rundungen und weist stellenweise Hinterschneidungen bzw. Überhänge 8 auf.

Durch diese unebene Struktur ergeben sich zwischen den Vertiefungen 7 krallenähnliche Vorsprünge 6, welche sich rutschfest an Gegenflächen, an welchen der Ballon plaziert werden soll, anlegen können. Mit diesen Krallen ist es auch möglich, Partikelchen, wie Blutgerinsel od.dgl., aus Organen, Gefäßen od.dgl. des zu behandelnden menschlichen oder auch tierischen körper durch Ziehen des Ballonkatheters zu entfernen. Soll der Ballon im Körper des Patienten dauerhaft verbleiben, fördern bzw. erleichtern sie Einwachsen des Ballons in das Gewebe.

## Patentansprüche

1. Ballonkatheter mit einer aus dehnbarem Material bestehenden Ballonhülle, deren äußere Oberfläche eine unregelmäßig unebene Struktur hat, dadurch gekennzeichnet, daß die unebene Oberfläche der Ballonhülle (2) kraterartige Vertiefungen (7) von 0,1 bis 0,5 mm, vorzugsweise mindestens 0,3 mm, Tiefe hat.

2. Ballonkatheter nach Anspruch 1, dadurch gekennzeichnet, daß die unebene Oberfläche Hinterschneidungen bzw. Überhänge (8) hat.

3. Ballonkatheter nach Anspruch 1, dadurch gekennzeichnet, daß die unebene Oberfläche Kanten, Spitzen und Rundungen hat.

4. Ballonkatheter nach Anspruch 1, dadurch gekennzeichnet, daß die unebene Oberfläche krallenförmige Erhöhungen (6) hat.

5. Ballonkatheter mit einer aus dehnbarem Material bestehenden Ballonhülle, deren äußere Oberfläche eine unregelmäßig unebene Struktur hat, die von einer aus dehnbarem Material bestehenden Beschichtung (5) gebildet ist, die auf der eigentlichen Ballonhülle (2) sitzt, dadurch gekennzeichnet, daß die unebene Oberfläche eine schaumförmige Struktur mit offenen Poren hat.

6. Ballonkatheter nach Anspruch 6, dadurch gekennzeichnet, daß die Beschichtung (5) aus Latex, Silikon, Polyurethan od. dgl. besteht.

## Claims

1. Balloon catheter with a balloon envelope made of extensible material, whose outer surface has an irregularly uneven structure, characterised in that the uneven surface of the balloon envelope (2) has crater-like recesses (7) with depth of 0.1 to 0.5 mm, preferably at least 0.3 mm.

2. Balloon catheter according to claim 1, characterised in that the uneven surface has undercut or projecting portions (8).

3. Balloon catheter according to claim 1, characterised in that the uneven surface has edges, points and curves.

4. Balloon catheter according to claim 1, characterised in that the uneven surface has claw-like protrusions (6).

5. Balloon catheter with a balloon envelope made of extensible material, whose outer surface has an irregularly uneven structure which consists of a coating (5) of extensible material which is seated on the actual balloon envelope (2), characterised in that the uneven surface has a foam-like structure with open pores.

6. Balloon catheter according to claim 6, characterised in that the coating (5) is made of latex, silicone, polyurethane or the like.

## Revendications

1. Cathéter à ballonnet ayant une enveloppe de ballonnet faite d'une matière étirable, dont la surface extérieure a une structure inégale irrégulièrement caractérisé en ce que la surface inégale de l'enveloppe de ballonnet (2) a des dépressions (7) analogues à des cratères d'une profondeur de 0,1 à 0,5 mm, avantageusement de 0,3 mm au moins.

2. Cathéter à ballonnet selon la revendication 1, caractérisé en ce que la surface inégale a des excavations ou des surplombs (8).

3. Cathéter à ballonnet selon la revendication 1, caractérisé en ce que la surface inégale a des arête, des pointes et des arrondis.

4. Cathéter à ballonnet selon la revendication 1, caractérisé en ce que la surface inégale a des parties saillantes (6) analogues à des griffes.

5. Cathéter à ballonnet ayant une enveloppe de ballonnet faite d'une matière étirable dont la surface extérieure a une structure inégale irrégulièrement constituée par un revêtement (5) fait de matière étirable appliquée sur la paroi propre du ballonnet (2) caractérisé en ce que la surface inégale a une structure analogue à celle d'une mousse à pores ouverts.

6. Cathéter à ballonnet selon la revendication 6, caractérisé en ce que le revêtement (5) est en latex, en silicone, en polyuréthane ou en matière similaire.

FIG.1

FIG.2

EP 0 204 218 B1